# EUROPÄISCHE PATENTSCHRIFT

(11) **EP 4 447 871 B1**
(45) Veröffentlichungstag und Bekanntmachung des Hinweises auf die Patenterteilung: **16.04.2025**
(21) Anmeldenummer: 22836072.3
(22) Anmeldetag: 13.12.2022
(51) Int. Cl.: A61F 2/50, A61F 2/76

(54) **PROTHESENSCHAFT**
PROSTHETIC SOCKET
EMBOÎTURE PROTHÉTIQUE

(30) Priorität: 16.12.2021 DE 102021133404
(43) Veröffentlichungstag der Anmeldung: 23.10.2024
(73) Patentinhaber: Ottobock SE & Co. KGaA, 37115 Duderstadt (DE)
(72) Erfinder: FINKE, Lars Benjamin, 37115 Duderstadt (DE)
(74) Vertreter: Gramm, Lins & Partner Patent- und Rechtsanwälte PartGmbB
(86) Internationale Anmeldenummer: PCT/EP2022/085490
(87) Internationale Veröffentlichungsnummer: WO 2023/110797

(56) Entgegenhaltungen:
- US-A1- 2019 183 663
- US-A1- 2021 113 356

## Beschreibung

Die Erfindung betrifft einen Prothesenschaft, der ein offenes proximales Ende, ein distales Ende mit einer distalen Anlagefläche und eine Mantelfläche aufweist, die sich zwischen dem proximalen Ende und dem distalen Ende erstreckt, wobei sich die wirksame Länge des Prothesenschaftes von der distalen Anlagefläche des distalen Endes bis zum proximalen Ende erstreckt, wobei ein wirksamer Umfang des Prothesenschaftes entlang der Innenfläche der Mantelfläche verläuft.

Ein derartiger Prothesenschaft ist beispielsweise aus der DE 10 2010 019 843 A1 bekannt. Der Prothesenschaft, der als Teil einer Prothese verwendet wird, verfügt über ein offenes proximales Ende, in das ein Amputationsstumpf eingeführt wird, wenn die Prothese, deren Teil der Prothesenschaft ist, vom Träger der Prothese getragen werden soll. Am geschlossenen distalen Ende des Prothesenschaftes ist eine Protheseneinrichtung, beispielsweise ein Kniegelenk, ein Unterschenkelrohr oder ein Prothesenfuß oder eine andere Art von Prothese angeordnet. Prothesenschäfte werden sowohl für Prothesen der unteren Extremität als auch für Prothesen der oberen Extremität verwendet. Um eine besonders gute Haftung des Prothesenschaftes am Amputationsstumpf und gegebenenfalls eine Polsterung zu erreichen, ist der Amputationsstumpf in der Regel von einem Liner umhüllt, der aus einem elastischen Material, beispielsweise Silikon, hergestellt wird.

Der Amputationsstumpf liegt an der distalen Anlagefläche des Prothesenschaftes an, wobei sich ein Liner zwischen dem Amputationsstumpf und der distalen Anlagefläche befinden kann. Die distale Anlagefläche wird durch ein separates Element oder Bauteil gebildet. Das distale Ende ist vorzugsweise geschlossen ausgebildet. In diesem Fall ist die distale Anlagefläche als eine Art doppelter Boden parallel zum distalen Ende des Schaftes angeordnet. Das distale Ende des Prothesenschaftes kann auch offen ausgestaltet sein.

Insbesondere bei Prothesen der unteren Extremität, beispielsweise Oberschenkelprothesen oder Unterschenkelprothesen trägt die Prothese bei jedem Schritt, den der Träger der Prothese geht oder läuft, das Gesamtgewicht des Trägers und ist daher hohen mechanischen Belastungen ausgesetzt. Gleiches gilt für den sich im Prothesenschaft befindenden Amputationsstumpf. Es ist daher sehr wichtig, dass der Prothesenschaft besonders gut an die geometrische Form des Amputationsstumpfes angepasst ist, um Druckstellen und damit Wunden und Schmerzen zu vermeiden. Problematisch ist dabei, dass insbesondere in der ersten Zeit nach der Amputation die Form des Amputationsstumpfes Veränderungen unterworfen ist, da sich insbesondere im Amputationsstumpf befindenden Muskeln aufgrund der Inaktivierung zurück bilden und sich Narben und Gewebeverhärtungen ausbilden. Im Laufe eines Tages verändert sich zudem das Stumpfvolumen durch Flüssigkeitsumlagerungen im Gewebe.

Um diesen Änderungen im Volumen des Amputationsstumpfes und in dessen Form Rechnung zu tragen, ist es aus dem Stand der Technik bekannt, sowohl den wirksamen Umfang des Prothesenschaftes als auch die wirksame Länge des Prothesenschaftes veränderbar auszugestalten. Die DE 10 2010 019 843 A1 verfügt dafür über eine Spanneinrichtung, die beispielsweise über ein Zugseil oder einen Draht verfügt, über den eine Spannung auf die verschiedenen Elemente des Prothesenschaftes ausgeübt werden kann. Dadurch lässt sich der wirksame Umfang des Prothesenschaftes auch für den Benutzer des Prothesenschaftes selbst einstellen. Zusätzlich verfügt der dort beschriebene Schaft über eine Einstelleinrichtung, über die die wirksame Länge des Prothesenschaftes, der im Stand der Technik auch als Hülse bezeichnet wird, eingestellt werden kann.

Nachteilig ist, dass trotz dieser Einstellbarkeit der wirksamen Länge und des wirksamen Umfanges die Passform des Prothesenschaftes durch den Träger der jeweiligen Prothese in nicht optimaler Weise angepasst und so Schmerzen und Probleme hervorgerufen werden können. So ist es für den Benutzer und Träger einer Prothese oft nicht oder zumindest nicht leicht zu erkennen, ob einer Abnahme des Volumens des Amputationsstumpfes durch die Verringerung des wirksamen Umfangs, die Verringerung der wirksamen Länge oder beider Maßnahmen Rechnung getragen werden soll. Gleiches gilt für eine Vergrößerung des Volumens des Amputationsstumpfes, wie sie im Laufe eines Tages vorkommt. Vergrößert der Träger der Prothese den wirksamen Umfang des Prothesenschaftes, kann dies dem durch die Volumenzunahme entstandenen hohen Druckes, der auf den Amputationsstumpf wirkt, entgegenwirken und Linderung bringen. Gleichzeitig kann dies jedoch zur Folge haben, dass die Gewichts- und Kräfteverteilung innerhalb des Prothesenschaftes nicht mehr optimal ist, sodass es zu Druckstellen und Schmerzen kommen kann. Umgekehrt kann die zu starke Vergrößerung der wirksamen Länge des Prothesenschaftes dazu führen, dass das distale Ende des Amputationsstumpfes den Kontakt mit der Innenfläche des distalen Endes des Prothesenschaftes verliert. Auch dabei kann die Kräfteverteilung innerhalb des Systems aus Prothesenschaft und Amputationsstumpf so verändert werden, dass es zu Druckstellen und Schmerzen kommt. Es besteht insbesondere die Gefahr von Ödembildungen am distalen Ende des Amputationsstumpfes.

Die US 2019/183 663 A1 offenbart einen Prothesenschaft mit einem Aktuator, über den der Innenumfang des Prothesenschaftes veränderbar ist, und einem Sensor, der mit einer Steuerungseinrichtung gekoppelt ist, die mit dem Aktuator verbunden ist und diesen in Abhängigkeit von den empfangenen Sensorsignalen aktiviert oder deaktiviert. US 2019/183 663 A1 zeigt die Merkmale des Oberbegrifs des Anspruchs 1.

Der Erfindung liegt daher die Aufgabe zugrunde, die Nachteile aus dem Stand der Technik zu beheben oder zumindest zu lindern.

Die Erfindung löst die gestellte Aufgabe durch einen Prothesenschaft gemäß dem Oberbegriff des Anspruchs 1, der sich dadurch auszeichnet, dass der Prothesenschaft eine elektrische Steuerung und eine Einstelleinrichtung für die wirksame Länge und den wirksamen Umfang aufweist und wenigstens einen ersten Sensor zum Erfassen einer distal auf den Amputationsstumpf wirkenden Kraft und wenigstens einen zweiten Sensor zum Erfassen von radial auf den Amputationsstumpf wirkenden Kräften aufweist, deren Messwerte an die elektrische Steuerung übermittelt werden, wobei die elektrische Steuerung eingerichtet ist, in Abhängigkeit der Messwerte beider Sensoren die Einstelleinrichtung zu steuern.

Der erfindungsgemäße Prothesenschaft verfügt über eine Einstelleinrichtung, mit der die wirksame Länge und der wirksame Umfang des Prothesenschaftes eingestellt und verändert werden kann. Die Einstelleinrichtung wird über die elektrische Steuerung des Prothesenschaftes, die beispielsweise eine elektronische Datenverarbeitungseinrichtung sein kann, gesteuert. So können vorzugsweise der wirksame Umfang und die wirksame Länge jeweils auf die individuellen Bedürfnisse des Trägers der Prothese und die aktuelle Situation des Amputationsstumpfes eingestellt werden.

Der Prothesenschaft verfügt über einen ersten Sensor zum Erfassen einer distal auf den Amputationsstumpf wirkenden Kraft und wenigstens einen zweiten Sensor zum Erfassen von radial auf den Amputationsstumpf wirkenden Kräften Vorzugsweise weisen der erste Sensor und/oder der zweite Sensor einen Drucksensor, einen Kraftsensor, einen Momentensensor und/oder einen Verformungssensor auf. Im Sinne der vorliegenden Anmeldung kann ein Drucksensor einen Druck erfassen, wenn sich aus den Messwerten des Drucksensors Rückschlüsse auf den herrschenden Druck ziehen lassen. Es ist nicht notwendig, dass der Drucksensor direkt den Druck misst. Entsprechend wird unter einem Kraftsensor jeder Sensor verstanden, aus dessen Messdaten Rückschlüsse auf eine Kraft gezogen werden können. Ein Momentensensor ist im Rahmen der vorliegenden Erfindung jeder Sensor, dessen Messwerte Rückschlüsse auf ein Moment erlauben, und ein Verformungssensor ist jeder Sensor, aus dessen Messwerten Aussagen über eine Verformung des Amputationsstumpfes getroffen werden können.

Als erster Sensor und/oder zweiter Sensor können beispielsweise hydraulisch oder pneumatisch arbeitende Systeme verwendet werden. Bei einem hydraulischen oder einem pneumatischen System wird beispielsweise der Druck des Hydraulikfluids oder des Gases, das sich in dem hydraulischen oder pneumatischen System befindet, gemessen. Er ist ein Maß für den Druck, der von außen, beispielsweise durch den Amputationsstumpf und/oder den Prothesenschaft auf das System aufgebracht wird. Das Arbeitsmedium eines pneumatischen Systems ist vorzugsweise ein kompressibles Gas, das Arbeitsmedium eines hydraulischen Systems ist vorzugsweise ein inkompressibles Fluid, beispielsweise eine Flüssigkeit, vorzugsweise ein Hydrauliköl.

Als erster und/oder zweiter Sensor kann beispielsweise ein optischer Sensor verwendet werden, der mittels optischer Messung den wirkenden Druck misst. Dazu kann beispielsweise ein drucksensibles Element, beispielsweise ein Federelement verwendet werden, das durch den Amputationsstumpf mehr oder weniger stark ausdehnt oder komprimiert wird oder auf andere Weise verformt oder ausgelenkt wird. Diese Ausdehnung, Kompression, Verformung oder Auslenkung kann dann durch den optischen Sensor detektiert werden.

Ein Verformungssensor kann beispielsweise ein kapazitiv und/oder resistiv wirkender Sensor sein. Bei einem kapazitiven Sensor bilden beispielsweise die Außenseite des Amputationsstumpfes oder eines darüber gezogenen Liners einerseits und die Innenfläche des Prothesenschaftes andererseits die beiden Platten eines elektrischen Kondensators. Der Abstand der beiden so gebildeten Platten bestimmt die Kapazität des Kondensators, die gemessen wird. Eine Abstandsänderung und damit eine Verformung des Amputationsstumpfes verändert die Kapazität des Kondensators. So kann der Abstand indirekt über die Kapazität des Kondensators bestimmt werden.

Bei einem resistiven Sensor verändert eine Verformung des Amputationsstumpfes einen elektrischen Widerstand, der gemessen wird. Eine Änderung des Widerstandes enthält folglich Informationen über eine Verformung des Amputationsstumpfes.

Alternativ oder zusätzlich dazu kann als erster Sensor und/oder als zweiter Sensor ein Dehnungsmessstreifen oder eine Hall-Sonde verwendet werden, um eine Verformung des Amputationsstumpfes zu erfassen.

Alternativ oder zusätzlich dazu kann als erster Sensor und/oder als zweiter Sensor ein Dehnungsmessstreifen verwendet werden, um die Verformung eines Teiles der Einstelleinrichtung des Prothesenschaftes zu erfassen. Weiterhin kann ein Dehnungsmessstreifen in Kombination mit einer Wägezelle genutzt werden um eine auf den Stumpf ausgeübte Kraft zu erfassen.

Wichtig ist unabhängig von der Wahl der Sensoren, dass ein erster Sensor und ein zweiter Sensor verwendet werden, deren Messwerte zur Steuerung der Einstelleinrichtung verwendet werden.

Der erste Sensor und der zweite Sensor senden ihre Messwerte in Form von Messsignalen an die elektrische Steuerung. Diese ist eingerichtet, in Abhängigkeit dieser Messwerte zu ermitteln, ob und wenn ja wie die wirksame Länge und der wirksame Umfang des Prothesenschaftes angepasst werden muss. Zudem ist die elektrische Steuerung eingerichtet, Steuersignale zu erzeugen und an die Einstelleinrichtung zu übermitteln. Die Einstelleinrichtung wird durch die Steuersignale derart angesteuert, dass die wirksame Länge und der wirksame Umfang entsprechend angepasst werden.

Bei einem erfindungsgemäßen Prothesenschaft ist es nicht zwangsläufig nötig, dass die wirksame Länge und der wirksame Umfang immer gleichzeitig verändert werden. Es ist durchaus möglich, dass für bestimmte Situation die elektrische Steuerung anhand der ihr übermittelten Messwerte erkennt, dass nur einer der beiden, beispielsweise der wirksame Umfang oder die wirksame Länge, zu verändern sind. Unabhängig davon, ob die wirksame Länge oder der wirksame Umfang oder beide Größen zu verändern sind, sind jeweils der erfasste distale Druck unter erfasste radiale Druck Eingangsgrößen, auf deren Grundlage die elektrische Steuerung ihre Algorithmen und Verfahren durchführt und die Steuersignale generiert. Es ist bei einem erfindungsgemäßen Prothesenschaft nicht möglich, dass die elektrische Steuerung die Einstelleinrichtung steuert, ohne dass sowohl der distale Druck als auch der radiale Druck erfasst und an die elektrische Steuerung übermittelt wurden.

Vorzugsweise verfügt die Einstelleinrichtung über ein bewegbares Endelement, durch das die wirksame Länge des Prothesenschaftes veränderbar ist, und/oder über wenigstens ein bewegbares Umfangselement, durch das der wirksame Umfang veränderbar ist. Das bewegbare Endelement bildet dann vorzugsweise die distale Anlagefläche.

**In** einer bevorzugten Ausgestaltung wird durch die Veränderung der wirksamen Länge und des wirksamen Umfanges eine Veränderung des distalen Druckes und des radialen Druckes erreicht, was zu einer Veränderung der Messwerte der beiden Sensoren führt. Auf diese Weise lässt sich eine Regelschleife erzeugen. Vorzugsweise greift die elektrische Steuerung auf einen elektronischen Datenspeicher zu, in dem Sollwerte für die Messwerte der beiden Sensoren, vorzugsweise für den distalen Druck und den radialen Druck hinterlegt sind. Die elektrische Steuerung ist eingerichtet, diesen hinterlegten Sollwert, beispielsweise einen hinterlegten distalen Druck und radialen Druck und die Messwerte der Sensoren so zu verarbeiten, dass sie miteinander vergleichbar sind, sodass die elektrische Steuerung aus diesem Vergleich ermittelt, ob und in welche Richtung der wirksame Umfang und die wirksame Länge verändert werden müssen.

Vorzugsweise bildet das bewegbare Endelement die distale Anlagefläche des distalen Endes des Prothesenschaftes. Um also die wirksame Länge des Prothesenschaftes zu verändern, wird in diesem Fall das bewegbare Endelement in Richtung auf das offene proximale Ende des Prothesenschaftes oder von ihm weg bewegt. Um die wirksame Länge des Prothesenschaftes zu reduzieren, wird das bewegbare Endelement in Richtung auf das proximale Ende des Prothesenschaftes bewegt. Eine Bewegung in die umgekehrte Richtung, also vom proximalen Ende des Prothesenschaftes weg, führt zu einer Vergrößerung der wirksamen Länge des Prothesenschaftes.

Vorzugsweise ist das wenigstens eine bewegbare Umfangselement so angeordnet, dass es wenigstens einen Teil der Innenfläche der Mantelfläche des Prothesenschaftes bildet. Besonders bevorzugt ist das wenigstens eine bewegbare Umfangselement in einer Ausnehmung des Grundkörpers des Prothesenschaftes angeordnet. **In** diesem Fall lässt sich der wirksame Umfang des Prothesenschaftes auf besonders einfache Weise dadurch verringern, dass das wenigstens eine bewegbare Umfangselement in der Ausnehmung des Grundkörpers des Prothesenschaftes nach radial innen, also in Richtung auf die Längsachse des Prothesenschaftes, entlang derer sich auch die wirksame Länge des Prothesenschaftes erstreckt, bewegt wird. Um den wirksamen Umfang des Prothesenschaftes zu vergrößern, wird das wenigstens eine bewegbare Umfangselement in dieser Ausgestaltung in die entgegengesetzte Richtung, also nach radial außen in Bezug auf die Längsachse des Prothesenschaftes, bewegt.

Alternativ oder zusätzlich dazu verfügt der Prothesenschaft über mehrere Umfangselemente, die überlappend angeordnet sind und die gemeinsam zumindest einen Teil des wirksamen Umfangs, bevorzugt jedoch den gesamten wirksamen Umfang des Prothesenschaftes bilden. Diese Ausgestaltung wird oft als "Tulpe" bezeichnet. Der Prothesenschaft verfügt in diesem Fall vorzugsweise über eine Spanneinrichtung, beispielsweise in Form eines spannbaren Drahtes oder Seilzuges, durch den der Grad der Überlappung der verschiedenen Umfangselemente eingestellt und verändert werden kann. Soll der wirksame Umfang des Prothesenschaftes in dieser Ausführungsform reduziert werden, wird die Spannung auf den Draht oder Seilzug erhöht und die Umfangselemente, die beweglich ausgebildet sind, so bewegt, dass sie einander zu einem größeren Anteil überlappen. Dadurch wird der wirksame Umfang des Prothesenschaftes reduziert. Umgekehrt kann durch eine Reduzierung der Spannung auf den Draht unter den Seilzug erreicht werden, dass sich die bewegbar ausgebildeten Umfangselemente so bewegen, dass sie einander zu einem geringeren Anteil überlappen, wodurch der wirksame Umfang des Prothesenschaftes vergrößert wird. Selbst verständlich auch eine Kombination der unterschiedlichen Ausgestaltungen, beispielsweise mehrerer einander überlappender Umfangselemente und wenigstens einem in einer Ausnehmung des Grundkörpers positioniert Umfangselement möglich.

Der Prothesenschaft verfügt vorzugsweise über wenigstens einen Antrieb, der das bewegbare Endelement bewegt. Der Prothesenschaft verfügt über wenigstens einen Antrieb, der das wenigstens eine bewegbare Umfangselement bewegt. **In** einer möglichen Ausführungsform werden die Bewegungen durch den gleichen Antrieb vorgenommen, sodass der Prothesenschaft nur über einen einzigen Antrieb verfügt. Der oder die Antriebe sind dabei Teile der Einstelleinrichtung.

**In** einer bevorzugten Ausgestaltung des Prothesenschaftes erfasst der erste Sensor eine von einem in den Prothesenschaft eingeführten Amputationsstumpf auf das bewegliche Endelement ausgeübte Kraft. Der zweite Sensor erfasst eine von einem in den Prothesenschaft eingeführten Amputationsstumpf auf das bewegliche Umfangselement ausgeübte Kraft. Die elektrische Steuerung, vorzugsweise eine elektronische Datenverarbeitungseinrichtung, ist eingerichtet, die Einstelleinrichtung, also beispielsweise das bewegbare Endelement und/oder das wenigstens eine bewegbare Umfangselement in Abhängigkeit der Messwerte der Sensoren zu bewegen. Besonders bevorzugt werden in der elektrischen Steuerung die von den Sensoren detektierten Werte mit jeweils einem Sollwert oder einem Sollwert-Bereich verglichen.

Die in der elektrischen Steuerung hinterlegten Sollwerte können sowohl Absolutwerte, als auch anzustrebende Verhältnisse der detektierten Kräfte auf den Amputationsstumpf zueinander beinhalten. Die Zielvorgabe ist es dabei grundsätzlich eine möglichst gleichmäßige vom Prothesenschaft auf den Amputationsstumpf ausgeübte Druckverteilung zu erhalten. Diese unterliegt jedoch anwenderspezifischen individuellen Schwankungen und wird vorzugsweise für jeden Patienten ermittelt und geprüft.

Ist eine detektierte Kraft an einem der beweglichen Elemente beispielsweise größer als ein vorbestimmter Sollwert, bewegt die elektrische Steuerung das bewegbare Element im Sinne einer verminderten Druckbelastung vom Amputationsstumpf weg. Ist eine detektierte Kraft an einem der beweglichen Elemente beispielsweise kleiner als ein vorbestimmter Sollwert, bewegt die elektrische Steuerung das bewegbare Element derart, dass der Druck erhöht wird. Dazu wird das jeweilige bewegbare Element beispielsweise auf den Amputationsstumpf zu bewegt. Besonders bevorzugt wird sowohl das bewegbare Endelement als auch das wenigstens eine bewegbare Umfangselement entsprechend simultan bewegt. Gegebenenfalls ist es jedoch ausreichend, nur das Endelement oder nur das Umfangselement zu bewegen um die gewünschte Druckverteilung zu erreichen. Die elektrische Steuerung sendet dazu beispielsweise Steuersignale an den Antrieb, beispielsweise einen Elektromotor, durch den das jeweilige Element, also das Endelement und/oder das Umfangselement, bewegt wird.

Ist der detektierte Druck an beiden Drucksensoren beispielsweise kleiner als der vorbestimmte Sollwert für den jeweiligen Sensor, bewegt die elektrische Steuerung das bewegbare Endelement und/oder das wenigstens eine bewegbare Umfangselement derart, dass das Volumen innerhalb des Prothesenschaftes abnimmt. Ist der detektierte Druck an beiden Drucksensoren hingegen größer als der vorbestimmte Sollwert für den jeweiligen Sensor, bewegt die elektrische Steuerung das bewegbare Endelement und/oder das wenigstens eine bewegbare Umfangselement derart, dass das Volumen innerhalb des Prothesenschaftes zunimmt. Der Bereich zwischen dem ersten Sollwert und dem vorbestimmten zweiten Sollwert kann als Sollwert-Bereich bezeichnet und angesehen werden.

Eine Kopplung der beiden Bewegungen des bewegbare Endelementes und des bewegbaren Umfangselementes erfolgt vorzugsweise durch die Verwendung eines mechatronischen Regelungssystems, zu dem die elektrische Steuerung und der wenigstens eine Antrieb gehören.

Besonders bevorzugt verfügt der Prothesenschaft über wenigstens einen zusätzlichen Sensor, vorzugsweise einen Feuchtigkeitssensor, einen Temperatursensor und/oder einen Sauerstoffsättigungssensor, und die elektrische Steuerung ist eingerichtet, das bewegbare Endelement und/oder das bewegbare Umfangselement in Abhängigkeit der Messwerte des ersten und zweiten Sensors sowie dieses wenigstens einen zusätzlichen Sensors zu bewegen. Vorzugsweise ist der Feuchtigkeitssensor so angeordnet, dass er die Feuchtigkeit zwischen dem Prothesenschaft und dem Amputationsstumpf vorzugsweise zwischen dem Prothesenschaft und einem Liner, dem sich der Amputationsstumpf befindet, misst. Handelt sich bei dem zusätzlichen Sensor um einen Temperatursensor, ist dieser vorzugsweise so angeordnet, dass die Temperatur innerhalb des Prothesenschaftes, also vorzugsweise in dem Zwischenraum zwischen Prothesenschaft und Amputationsstumpf, gemessen wird. Ein Sauerstoffsättigungssensor ist vorzugsweise so angeordnet, dass er die Sauerstoffsättigung des Blutes innerhalb des Amputationsstumpfes bestimmen kann.

Vorzugsweise ist der wenigstens eine zusätzliche Sensor eingerichtet, Sensordaten zu erfassen, aus denen Rückschlüsse auf den einen Bewegungszustand des Trägers des Prothesenschaftes gezogen werden können. Besonders bevorzugt weist der wenigstens eine zusätzliche Sensor wenigstens einen Inertialsensor auf, der vorzugsweise eingerichtet ist, eine Raumlage, eine Geschwindigkeit und oder eine Winkeländerung des Prothesenschaftes oder eines mit dem Prothesenschaft direkt oder indirekt verbundenen Bauteils zu bestimmen. So kann beispielsweise die elektrische Steuerung die Einstelleinrichtung so steuern, dass die wirksame Länge und/oder der wirksame Umfang des Prothesenschaftes zunehmen, wenn der Träger des Prothesenschaftes sitzt. **In** diesem Fall werden keine oder zumindest keine regelmäßig wiederkehrenden Bewegungen des Prothesenschaftes detektiert. Auch ist der Prothesenschaft nicht so stark oder sogar gar nicht belastet.

Mithilfe der beiliegenden Figuren wird nachfolgend ein Ausführungsbeispiel der vorliegenden Erfindung näher erläutert. Es zeigt
Figur 1 - die schematische Darstellung verschiedener Teile eines Prothesenschaftes und
Figur 2 - ein schematisches Ablaufdiagramm, das die Funktionsweise der elektrischen Steuerung darstellt.

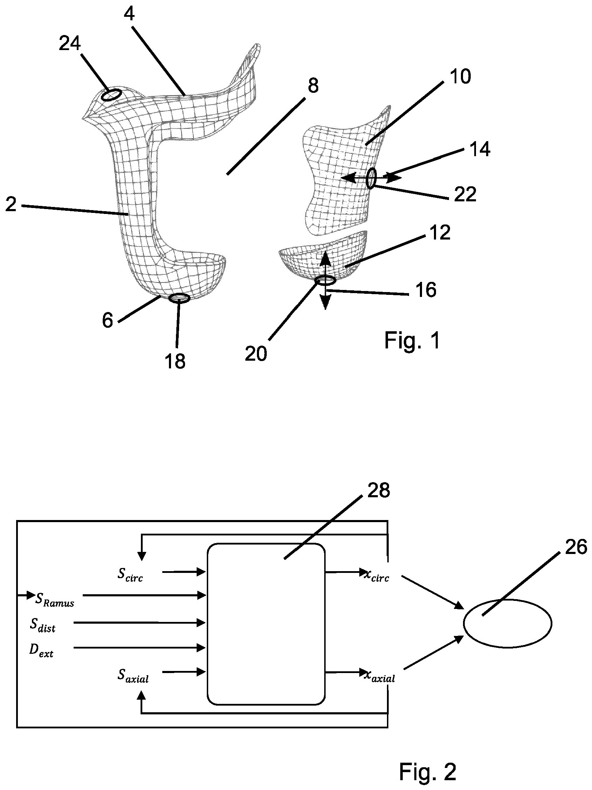

Figur 1 zeigt schematisch verschiedene Bauteile eines Prothesenschaftes gemäß einem ersten Ausführungsbeispiel der vorliegenden Erfindung. Der Prothesenschaft verfügt über einen Grundkörper 2, der ein offenes proximales Ende 4 und ein im gezeigten Ausführungsbeispiel geschlossenes distales Ende 6 aufweist. Er verfügt zudem über eine Ausnehmung 8, in der ein Umfangselement 10 angeordnet ist, das relativ zum Grundkörper 2 beweglich ist. Am distalen Ende 6 des Grundkörpers 2 befindet sich ein bewegliches Endelement 12, das in Figur 1 neben dem Grundkörper 2 dargestellt ist. Das bewegliche Umfangselement 10 ist entlang des radialen Doppelpfeiles 14 und das bewegliche Endelement 12 entlang des axialen Doppelpfeils 16 bewegbar. Die Begriffe "axial" und "radial" beziehen sich dabei auf die Längserstreckung des Prothesenschaftes, die sich entlang der wirksamen Länge des Prothesenschaftes von der Innenfläche des distalen Endes 6 bis zum proximalen Ende 4 erstreckt.

Am distalen Ende 6 des Grundkörpers 2 ist im gezeigten Ausführungsbeispiel ein distaler Sensor 18 angeordnet. Dieser misst einen axialen Druck oder eine axiale Kraft. Diese wird als Distalkraft bezeichnet und ist die resultierende Gesamtkraft an einem Schaftadapter, der in Figur 1 aus Übersichtlichkeitsgründen nicht dargestellt ist. An dem Schaftadapter können weitere Prothesenelemente, im gezeigten Ausführungsbeispiel einer Oberschenkelprothese beispielsweise ein Prothesenkniegelenk, angeordnet werden. Ein erster Sensor 20 ist am beweglichen Endelement 12 positioniert und misst den Druck oder eine Kraft, durch die das distale Ende eines Amputationsstumpfes belastet ist. Dieser Druck wird als axialer Druck bezeichnet. Der Prothesenschaft verfügt über einen zweiten Sensor 22, der im gezeigten Ausführungsbeispiel am beweglichen Umfangselement angeordnet ist und eine radiale Kraft oder einen radialen Druck misst. Ein Ramus-Drucksensor 24 misst die Kraft einer knöchernen Anlage des Prothesenschaftes und wird auch als Ramussensor bezeichnet.

Figur 2 zeigt schematisch, wie die einzelnen Daten oder Messwerte, die von den einzelnen Sensoren aufgenommen werden, verwendet werden können. Ziel ist eine möglichst optimale Auswahl der wirksamen Länge xₐₓᵢₐₗ und des wirksamen Umfanges x_{circ}, die durch die Verschiebung des beweglichen Endelementes 12 und des beweglichen Umfangselementes 10 verändert und angepasst werden können. Diese bilden die Zielkonfiguration 26. Dazu werden die Messwerte S_{circ} des zweiten Drucksensors 22, S_{Ramus} des Ramus-Drucksensors 24, S_{dist} des distalen Drucksensors 18 und Sₐₓᵢₐₗ des ersten Drucksensors 20 einer elektrischen Steuerung 28 zugeführt. Im gezeigten Ausführungsbeispiel werden zudem weitere Daten Dₑₓₜ beispielsweise von externen Sensoren, die in Figur 1 nicht dargestellt sind, und/oder beispielsweise manuelle Nutzervorgaben berücksichtigt und ebenfalls der elektrischen Steuerung 28 zugeleitet.

Die elektrische Steuerung 28 errechnet aus den ihr zur Verfügung bereitgestellten Daten und Messwerten Werte für den wirksamen Umfang x_{circ} und die wirksame Länge xₐₓᵢₐₗ des Prothesenschaftes und steuert einen Antrieb für die jeweilige Einstelleinrichtung derart, dass diese Werte eingestellt werden. Dadurch verändern sich die Messwerte der verschiedenen Sensoren und damit die Eingangswerte, die der elektrischen Steuerung 28 bereitgestellt werden, so lange, bis die Zielkonfiguration 26, die beispielsweise Sollwerte für die einzelnen Messgrößen enthält, erreicht ist.

### Bezugszeichenliste

- 2: Grundkörper
- 4: proximales Ende
- 6: distales Ende
- 8: Ausnehmung
- 10: Umfangselement
- 12: Endelement
- 14: radialer Doppelpfeil
- 16: axialer Doppelpfeil
- 18: distaler Sensor
- 20: erster Sensor
- 22: zweiter Sensor
- 24: Ramus-Drucksensor
- 26: Zielkonfiguration
- 28: Elektrische Steuerung

## Patentansprüche

1. Prothesenschaft, der
- ein offenes proximales Ende (4),
- ein distales Ende (6) mit einer distalen Anlagefläche und
- eine Mantelfläche aufweist, die sich zwischen dem proximalen Ende (4) und dem distalen Ende (6) erstreckt,
wobei sich eine wirksame Länge des Prothesenschaftes von der distalen Anlagefläche des distalen Endes (6) bis zum proximalen Ende (4) erstreckt,
wobei ein wirksamer Umfang des Prothesenschaftes entlang der Innenfläche der Mantelfläche verläuft,
**dadurch gekennzeichnet, dass** der Prothesenschaft eine elektrische Steuerung und eine Einstelleinrichtung für die wirksame Länge und den wirksamen Umfang aufweist und wenigstens einen ersten Sensor (20) zum Erfassen einer distal auf den Amputationsstumpf wirkenden Kraft und wenigstens einen zweiten Sensor (22) zum Erfassen von radial auf den Amputationsstumpf wirkenden Kräften aufweist, deren Messwerte an die elektrische Steuerung übermittelt werden, wobei die elektrische Steuerung eingerichtet ist, in Abhängigkeit der Messwerte beider Sensoren (20, 22) die Einstelleinrichtung zu steuern.

2. Prothesenschaft nach Anspruch 1, **dadurch gekennzeichnet, dass** der erste Sensor (20) und/oder der zweite Sensor (22) einen Drucksensor, einen Kraftsensor, einen Momentensensor und/der einen Verformungssensor aufweist.

3. Prothesenschaft nach Anspruch 1 oder 2, **dadurch gekennzeichnet, dass** die Einstelleinrichtung ein bewegbares Endelement (12), durch das die wirksame Länge veränderbar ist, und/oder wenigstens ein bewegbares Umfangselement (10) aufweist, durch das der wirksame Umfang veränderbar ist.

4. Prothesenschaft nach Anspruch 3, **dadurch gekennzeichnet, dass** das bewegbare Endelement (12) die distale Anlagefläche des distalen Endes (6) bildet.

5. Prothesenschaft nach Anspruch 3 oder 4, **dadurch gekennzeichnet, dass** das wenigstens eine bewegbare Umfangselement (12) zumindest einen Teil der Innenfläche der Mantelfläche bildet.

6. Prothesenschaft nach einem der vorstehenden Ansprüche, **dadurch gekennzeichnet, dass** das wenigstens eine Umfangselement (10) in einer Ausnehmung (8) eines Grundkörpers (2) des Prothesenschaftes angeordnet ist.

7. Prothesenschaft nach einem der vorstehenden Ansprüche, **dadurch gekennzeichnet, dass** der erste Drucksensor (20) eingerichtet und angeordnet ist, einen von einem in den Prothesenschaft eingeführten Amputationsstumpf auf das bewegliche Endelement (12) zu messen und/oder der zweite Drucksensor (22) eingerichtet und angeordnet ist, einen von einem in den Prothesenschaft eingeführten Amputationsstumpf auf das wenigstens eine bewegliche Umfangselement (10) ausgeübten Druck zu messen.

8. Prothesenschaft nach Anspruch 7, **dadurch gekennzeichnet, dass** der Prothesenschaft wenigstens einen zusätzlichen Sensor, vorzugsweise einen Feuchtigkeitssensor, Temperatursensor und/oder Sauerstoffsättigungssensor, aufweist und die elektrische Steuerung (28) eingerichtet ist, das bewegbare Endelement (12) und/oder das wenigstens eine bewegbare Umfangselement (10) in Abhängigkeit der Messwerte des wenigstens einen zusätzlichen Sensors zu bewegen.

9. Prothesenschaft nach Anspruch 8, **dadurch gekennzeichnet, dass** der wenigstens eine zusätzliche Sensor eingerichtet ist, Sensordaten zu erfassen, aus denen Rückschlüsse auf den einen Bewegungszustand des Trägers des Prothesenschaftes gezogen werden können.

10. Prothesenschaft nach Anspruch 9, **dadurch gekennzeichnet, dass** der wenigstens eine zusätzliche Sensor wenigstens einen Inertialsensor aufweist, der vorzugsweise eingerichtet ist, eine Raumlage, eine Geschwindigkeit und oder eine Winkeländerung des Prothesenschaftes oder eines mit dem Prothesenschaft direkt oder indirekt verbundenen Bauteils zu bestimmen.

## Claims

1. A prosthetic socket that comprises
- an open proximal end (4),
- a distal end (6) with a distal contact surface and
- a lateral surface that extends between the proximal end (4) and the distal end (6),
wherein an effective length of the prosthetic socket extends from the distal contact surface of the distal end (6) to the proximal end (4),
wherein an effective circumference of the prosthetic socket extends along the inner surface of the lateral surface,
**characterized in that** the prosthetic socket comprises an electrical control unit and an adjustment device for adjusting the effective length and the effective circumference, and at least one first sensor (20) for detecting a force acting distally on the amputation stump and at least one second sensor (22) for detecting forces acting radially on the amputation stump, the measured values of which are transmitted to the electrical control unit, the electrical control unit being configured to control the adjustment device according to the measured values of both sensors (20, 22).

2. The prosthetic socket according to claim 1, **characterized in that** the first sensor (20) and/or the second sensor (22) comprises a pressure sensor, a force sensor, a torque sensor and/or a deformation sensor.

3. The prosthetic socket according to claim 1 or 2, **characterized in that** the adjustment device comprises a moveable end element (12), by means of which the effective length can be changed, and/or at least one moveable circumferential element (10), by means of which the effective circumference can be changed.

4. The prosthetic socket according to claim 3, **characterized in that** the moveable end element (12) forms the distal contact surface of the distal end (6).

5. The prosthetic socket according to claim 3 or 4, **characterized in that** the at least one moveable circumferential element (12) forms at least part of the inner surface of the lateral surface.

6. The prosthetic socket according to one of the preceding claims, **characterized in that** the at least one circumferential element (10) is arranged in a recess (8) of a base body (2) of the prosthetic socket.

7. The prosthetic socket according to one of the preceding claims, **characterized in that** the first pressure sensor (20) is configured and arranged to measure a pressure exerted on the moveable end element (12) by an amputation stump inserted into the prosthetic socket and/or the second pressure sensor (22) is configured and arranged to measure a pressure exerted on the at least one moveable circumferential element (10) by an amputation stump inserted into the prosthetic socket.

8. The prosthetic socket according to claim 7, **characterized in that** the prosthetic socket has at least one additional sensor, preferably a moisture sensor, a temperature sensor and/or an oxygen saturation sensor, and the electrical control unit (28) is configured to move the moveable end element (12) and/or the at least one moveable circumferential element (10) depending on the measured values of the at least one additional sensor.

9. The prosthetic socket according to claim 8, **characterized in that** the at least one additional sensor is configured to detect sensor data from which conclusions can be drawn about the state of movement of the wearer of the prosthetic socket.

10. The prosthetic socket according to claim 9, **characterized in that** the at least one additional sensor has at least one inertial sensor that is preferably configured to determine a spatial position, a speed and/or a change in the angle of the prosthetic socket or of a component directly or indirectly connected to the prosthetic socket.

## Revendications

1. Emboîture de prothèse, comprenant
- une extrémité proximale (4) ouverte,
- une extrémité distale (6) ayant une surface d'appui distale, et
- une surface enveloppe s'étendant entre l'extrémité proximale (4) et l'extrémité distale (6),
une longueur efficace de l'emboîture de prothèse s'étendant depuis la surface d'appui distale de l'extrémité distale (6) jusqu'à l'extrémité proximale (4),
une circonférence efficace de l'emboîture de prothèse s'étendant le long de la face intérieure de la surface enveloppe,
**caractérisée en ce que** l'emboîture de prothèse comprend une commande électrique et un dispositif de réglage de la longueur efficace et de la circonférence efficace et comprend au moins un premier capteur (20) destiné à détecter une force agissant du côté distal sur le moignon d'amputation et au moins un deuxième capteur (22) destiné à détecter des forces agissant radialement sur le moignon d'amputation, dont les valeurs de mesure sont transmises à la commande électrique, la commande électrique étant conçue pour commander le dispositif de réglage en fonction des valeurs de mesure des deux capteurs (20, 22).

2. Emboîture de prothèse selon la revendication 1,
**caractérisée en ce que** le premier capteur (20) et/ou le deuxième capteur (22) comporte(nt) un capteur de pression, un capteur de force, un capteur de couple et/ou un capteur de déformation.

3. Emboîture de prothèse selon la revendication 1 ou 2,
**caractérisée en ce que** le dispositif de réglage comprend un élément d'extrémité mobile (12) qui permet de modifier la longueur efficace, et/ou au moins un élément circonférentiel mobile (10) qui permet de modifier la circonférence efficace.

4. Emboîture de prothèse selon la revendication 3,
**caractérisée en ce que** l'élément d'extrémité mobile (12) constitue la surface d'appui distale de l'extrémité distale (6).

5. Emboîture de prothèse selon la revendication 3 ou 4,
**caractérisée en ce que** ledit au moins un élément circonférentiel mobile (12) constitue au moins une partie de la face intérieure de la surface enveloppe.

6. Emboîture de prothèse selon l'une des revendications précédentes,
**caractérisée en ce que** ledit au moins un élément circonférentiel (10) est disposé dans un évidement (8) d'un corps de base (2) de l'emboîture de prothèse.

7. Emboîture de prothèse selon l'une des revendications précédentes,
**caractérisée en ce que** le premier capteur de pression (20) est conçu et agencé pour mesurer une pression exercée par un moignon d'amputation, inséré dans l'emboîture de prothèse, sur l'élément d'extrémité mobile (12) et/ou le deuxième capteur de pression (22) est conçu et agencé pour mesurer une pression exercée par un moignon d'amputation, inséré dans l'emboîture de prothèse, sur ledit au moins un élément circonférentiel mobile (10).

8. Emboîture de prothèse selon la revendication 7,
**caractérisée en ce que** l'emboîture de prothèse comprend au moins un capteur supplémentaire, de préférence un capteur d'humidité, un capteur de température et/ou un capteur de saturation en oxygène, et la commande électrique (28) est conçue pour déplacer l'élément d'extrémité mobile (12) et/ou ledit au moins un élément circonférentiel mobile (10) en fonction des valeurs de mesure dudit au moins un capteur supplémentaire.

9. Emboîture de prothèse selon la revendication 8,
**caractérisée en ce que** ledit au moins un capteur supplémentaire est conçu pour enregistrer des données de capteur permettant d'en déduire un état de mouvement du porteur de l'emboîture de prothèse.

10. Emboîture de prothèse selon la revendication 9,
**caractérisée en ce que** ledit au moins un capteur supplémentaire comprend au moins un capteur inertiel qui est de préférence conçu pour déterminer une position spatiale, une vitesse et/ou une variation angulaire de l'emboîture de prothèse ou d'un composant relié directement ou indirectement à l'emboîture de prothèse.
